# EUROPEAN PATENT APPLICATION

(11) **EP 3 431 506 A1**
(43) Date of publication of application: **23.01.2019**
(21) Application number: 17765747.5
(22) Date of filing: 07.03.2017
(51) Int. Cl.: C07K 19/00, C07K 14/00, C07K 1/02, C07K 1/04, C07K 1/06, A61K 38/16, A61P 35/00, A61P 11/00

(54) **MULTI-FUNCTIONAL FUSION POLYPEPTIDE, PREPARATION METHOD THEREOF, AND APPLICATION OF SAME**

(30) Priority: 14.03.2016 CN 201610144213
(71) Applicant: Nanjing Anji Biological Technology Co., Ltd, Nanjing, Jiangsu 210000 (CN)
(72) Inventor: XU, Hanmei, Nanjing Jiangsu 211100 (CN)
(74) Representative: Gong, Jinping
(86) International application number: PCT/CN2017/075861
(87) International publication number: WO 2017/157205

(57) **Abstract**

The invention discloses a multifunctional fusion polypeptide and its preparation method and application thereof, in the field of biopharmaceutics. The fusion polypeptide of the present invention comprises the domain Pro-(D-Pyr)-(D-Cys)-Bip-Arg-Gly-Glu, Ile-Val-Arg-Arg-Ala-Asp-Arg-Ala-Ala-Val-Pro, Arg-Gly-Asp, and Gly-Gly-Gly-Gly, and can treat human pulmonary fibrosis, lung tissue lesions, lung cancer and other tumors. In a cell model for pulmonary fibrosis, the polypeptide of the present invention can significantly lower the hydroxyproline content and suppress the progression of pulmonary fibrosis. MTT assay shows that the polypeptide of the present invention can inhibit the proliferation multiple human tumor cells. The polypeptide of the present invention is prepared by a synthetic method that is uncomplicated method and offers good application prospects.

## Description

### CROSS REFERENCE TO RELATED APPLICATION

This application is a national stage application of International application number PCT/CN2017/075861, filed March 7, 2017, titled "MULTI-FUNCTIONAL FUSION POLYPEPTIDE, PREPARATION METHOD THEREOF, AND APPLICATION OF SAME," which claims the priority benefit of Chinese Patent Application No. 201610144213.0, filed on March 14, 2016, which is hereby incorporated by reference in its entirety.

### TECHNICAL FIELD

This application relates in general to biopharmaceuticals and, in particular, to a
a novel multifunctional fusion polypeptide and its preparation method and application thereof.

### BACKGROUND

Pulmonary fibrosis, especially idiopathic pulmonary fibrosis (IPF), has long been considered as a progressive, largely irreversible pathological change. The 5-year mortality rate of IPF patients after diagnosis is 65%, a serious threat to public health. Because current treatment regiments have little effect, there is not even a consensus treatment plan for IPF. The most common symptom of pulmonary fibrosis is: difficulty breathing. In mild pulmonary fibrosis, dyspnea often occurs during periods of intense activity and is often overlooked or misdiagnosed as other diseases. When pulmonary fibrosis progresses, breathing difficulties occur at rest, and progressive pulmonary dysfunction can occur in patients with severe pulmonary fibrosis.

A variety of factors can cause pulmonary fibrosis, such as occupational dust inhalation, radiation damage and certain drugs (bleomycin). In addition, there is a class of unexplained pulmonary fibrosis (such as IPF). Although the causes are different, the development of pulmonary fibrosis is basically analogous, that is, it begins with the infiltration of inflammatory cells in the lower respiratory tract, which causes damage to alveolar epithelial cells and vascular endothelial cells, accompanied by the release of cytokines causing proliferation of myofibroblast (MF) and type II alveolar epithelial cells, which in turn leads to deposition of extracellular matrix proteins and collagen, which ultimately causes damage to the lung structure. Lung injury caused by various reasons has obvious inflammatory state at the initial stage, and the inflammatory state of pulmonary fibrosis is not obvious at the late stage, which may be an important factor for the loss of efficacy for anti-inflammatory therapy during the treatment of pulmonary fibrosis.

A recent study on the mechanism of pulmonary fibrosis found that the peptide Ile-Val-Arg-Arg-Ala-Asp-Arg-Ala-Ala-Val-Pro was a sequence of human angiogenesis endostatin that inhibits angiogenesis. The tripeptide Arg-Gly-Asp was found to specifically bind to the integrin protein expressed on the surface of neovascularization. The fusion peptide incorporating the above two peptides has been used for anti-tumor treatment and anti-rheumatoid treatment (Patent Publication No.: CN1699408A). The polypeptide Pro-(D-Pyr)-(D-Cys)-Bip-Arg-Gly-Glu (Patent Publication No.: CN102746380A) was found to inhibit the activity of extracellular matrix metalloproteinases. At present, the above described short peptides have been applied for the treatment of tumor and rheumatoid diseases, but they can only work towards a single target, and cannot simultaneously target multiple targets. Tumor cells are more prone to develop drug resistance against single-target drugs, compared to multi-target drugs.

### SUMMARY

In view of the problems of the existing polypeptide drugs including single-target, being prone to develop drug resistance, and lacking efficacy in treating pulmonary fibrosis, the present invention provides a multifunctional fusion polypeptide, and its preparation method thereof and application thereof. The multifunctional fusion polypeptide can effectively treat human pulmonary fibrosis, lung tissue lesions, lung cancer and other tumors. The multifunctional fusion polypeptide of the present invention comprises a plurality of domains targeting a plurality of targets. In the pulmonary fibrosis cell model, the multifunctional fusion polypeptide of the present invention can significantly reduce the hydroxyproline level in the model cells and inhibit the progression of pulmonary fibrosis.

In order to solve the problems described above, the invention discloses technical solutions in the follow.

A multifunctional fusion polypeptide, comprising a domain comprising Pro-(D-Pyr)-(D-Cys)-Bip-Arg-Gly-Glu, Ile-Val-Arg-Arg-Ala-Asp-Arg-Ala-Ala-Val-Pro, Arg-Gly-Asp, and Gly-Gly-Gly-Gly.

Preferably, the multifunctional fusion polypeptide has the amino acid sequence of peptide I: Pro-(D-Pyr)-(D-Cys)-Bip-Arg-Gly-Glu-Gly-Gly-Gly-Gly-Ile-Val-Arg-Arg-Ala-Asp-Arg-Ala-Ala -Val-Pro-Gly-Gly-Gly-Gly-Arg-Gly-Asp;

Preferably, the multifunctional fusion polypeptide has the amino acid sequence of peptide II: Ile-Val-Arg-Arg-Ala-Asp-Arg-Ala-Ala-Val-Pro-Gly-Gly-Gly-Gly-Pro-(D-Pyr)-(D-Cys) -Bip-Arg-Gly-Glu-Gly-Gly-Gly-Gly-Arg-Gly-Asp;

Preferably, the multifunctional fusion polypeptide has the amino acid sequence of peptide III: Arg-Gly-Asp-Gly-Gly-Gly-Gly-Pro-(D-Pyr)-(D-Cys)-Bip-Arg-Gly-Glu-Gly-Gly-Gly -Gly-Ile-Val-Arg-Arg-Ala-Asp-Arg-Ala-Ala-Val-Pro;

Preferably, the multifunctional fusion polypeptide has the amino acid sequence of peptide IV: Arg-Gly-Asp-Gly-Gly-Gly-Gly-Ile-Val-Arg-Arg-Ala-Asp-Arg-Ala-Ala-Val-Pro-Gly -Gly-Gly-Gly-Pro-(D-Pyr)-(D-Cys)-Bip-Arg-Gly-Glu;

Preferably, the multifunctional fusion polypeptide has the amino acid sequence of peptide V: Ile-Val-Arg-Arg-Ala-Asp-Arg-Ala-Ala-Val-Pro-Gly-Gly-Gly-Gly-Arg-Gly-Asp-Gly -Gly-Gly-Gly-Pro-(D-Pyr)-(D-Cys)-Bip-Arg-Gly-Glu;

Preferably, the multifunctional fusion polypeptide has the amino acid sequence of peptide VI: Pro-(D-Pyr)-(D-Cys)-Bip-Arg-Gly-Glu-Gly-Gly-Gly-Gly-Arg-Gly-Asp-Gly-Gly-Gly -Gly-Ile-Val-Arg-Arg-Ala-Asp-Arg-Ala-Ala-Val-Pro;

For the above peptide I-VI, Pyr is 3-(3-pyridyl)-L-alanine and Bip is L-4, 4'-biphenylalanine.

A method for using the multifunctional fusion polypeptides as described above, comprising, using the multifunctional fusion polypeptides to generate medicines efficacious in treating human pulmonary fibrosis, pulmonary tissue lesions, lung cancer, and tumor.

Preferably, the pulmonary fibrosis includes idiopathic pulmonary fibrosis and pulmonary fibrosis caused by occupational dust, radiation damage, and drug factors.

Preferably, the pulmonary tissue lesions include bacterial pneumonia, viral pneumonia, mycoplasmal pneumonia, chlamydia pneumonia, protozoal pneumonia and fungal pneumonia.

Preferably, the lung cancer comprises squamous cell carcinoma, adenocarcinoma, glandular scale cancer, small cell lung cancer, non-small cell lung cancer, and large cell carcinoma.

Preferably, the tumor comprises primary or secondary tumor, melanoma, hemangioma, and sarcoma originated from head, neck, brain, thyroid, esophagus, pancreas, liver, stomach, breast, kidney, gallbladder, colon or rectum, ovary, cervix, uterus, prostate, bladder or testis.

A method for preparing the multifunctional fusion polypeptide as disclosed in above, wherein the multifunctional fusion polypeptide is synthesized via a solid phase method or a liquid phase method.

Preferably, the solid-phase method includes the following steps: selecting a solid phase carrier such as Fmoc-wang-resin or Fmoc-CTC-resin as starting material, adding protected amino acid to the starting material, one-at-a-time, and according to the peptide sequence, to yield a 29-amino acid peptide. The 29-amino acid peptide is washed, detached from resin, and post-treated to yield a crude fusion polypeptide. The crude fusion polypeptide is dissolved, purified through a preparative high-performance liquid chromatography, and lyophilized to yield the fusion polypeptide.

Preferably, liquid phase method comprises the steps of sequentially connecting amino acids through an amide bond according to the fusion peptide sequence, wherein the inactive groups of the amino acids are protected by FMOC modification.

The present invention disclosed a fusion peptide that links three different short peptides having three different targets, the Gly-Gly-Gly-Gly linking sequence being optimized and flexible. The polypeptide sequence Arg-Gly-Asp can bind to Integrin ανβ3 on the cell surface and inhibit cell migration; the polypeptide sequence Ile-Val-Arg-Arg-Ala-Asp-Arg-Ala-Ala-Val-Pro can inhibit microvascular regeneration and neovascularization; the polypeptide sequence Pro-(D-Pyr)-(D-Cys)-Bip-Arg-Gly-Glu can inhibit the activity of extracellular matrix metalloproteinases and reduce the ability of cells to degrade extracellular matrices. By screening different combinations of short peptide sequences and amino acid linkage sequences, the fusion polypeptides disclosed in the present invention ensure that the binding of each amino acid domain to its target does not interfere with the binding of another amino acid domain to its respective target. The fusion polypeptide of the invention simultaneously targets multiple targets, and the fusion polypeptide of the invention can activate the apoptosis signal pathway of the tumor cell and improve the sensitivity of the tumor cell to the drug, and the anti-tumor effect is significantly better than any of the single-target peptide. In addition, the present invention disclosed an unexpected effect or function of fusing the three short peptides into one peptide, i.e., the significant reduction of the hydroxyproline level in the cells. The fusion polypeptide has a significant effect on the treatment of pulmonary fibrosis. Although the pathogenesis of pulmonary fibrosis is accompanied by abnormal angiogenesis and accumulation of extracellular matrix, existing drugs or polypeptides that inhibit angiogenesis and accumulation of extracellular matrix have no significant effect on the treatment of pulmonary fibrosis. In contrast, the fusion polypeptide of the present invention can significantly inhibit the progression of pulmonary fibrosis, and can also inhibit various pulmonary infections.

Compared with the prior art, the beneficial effects of the present invention include: (1) the fusion polypeptide of the present invention can be used for the treatment of pulmonary fibrosis, the composition is an amino acid, easy to synthesize, and has no obvious toxic and side effects; in the pulmonary fibrosis cell model, the fusion polypeptide of the present invention can significantly reduce hydroxyquinone content in the cell and improve lung fibrosis; (2) the fusion polypeptide of the invention has a significant inhibitory effect on pulmonary infection, and the highest inhibition rate is above 65%, which is superior to the effect of the penicillin group; and (3) the fusion polypeptide of the invention has an inhibitory effect on the growth of lung cancer cells, and the highest inhibition rate is over 93%, which has a better effect than docetaxel; (4) compared with docetaxel, the existing drug approved for the treatment of tumor, the polypeptide of the invention has better growth inhibition effect on various tumors, and has no obvious toxic and side effects; the preparation method of the polypeptide of the invention is simple and has good application prospects; (5) the fusion polypeptide of the present invention has an activity against lung fibrosis and anti-pulmonary infection as compared with the single-target peptides.

### BRIEF DESCRIPTION OF THE DRAWINGS

Figure 1 is a graph showing the reduction of hydroxyproline content in a pulmonary fibrosis cell model by polypeptides I and II of the present invention;
Figure 2 is a graph showing the reduction of hydroxyproline content in a pulmonary fibrosis cell model by polypeptide III and polypeptide IV of the present invention;
Figure 3 is a graph showing the reduction of hydroxyproline contentin a pulmonary fibrosis cell model by polypeptide V and polypeptide VI of the present invention;
Figure 4 is a graph showing the inhibitory effect of the polypeptides I, II, III, IV, V, and VI of the present invention on pulmonary infection;
Figure 5 is a graph showing the inhibitory effect of the polypeptides I, II, III, IV, V, VI of the present invention on the growth of lung cancer cells;
Figure 6 is a graph showing the inhibitory effects of the polypeptides I, II, III, IV, V, VI of the present invention on different types of tumor growth.

### DETAILED DESCRIPTION OF THE PREFERRED EMBODIMENTS

The invention is further described below in conjunction with specific embodiments.

### Example 1:

### Preparation and test of the multifunctional polypeptide

In the present embodiment, the polypeptides I-VI are synthesized by a solid phase synthesis method, and are separated and purified by preparative HPLC, and the purity of the polypeptide is determined by an analytical high-performance liquid chromatography.

The solid phase synthesis method for peptides I-VI is based on the solid phase carrier Fmoc-wang-resin (Jill Biochemical Co., Ltd.), wherein protected amino acids are sequentially added to form dipeptide, tripeptide, etc., until the 29-amino acid peptide terminal products. After all amino acids are added, the 29-amino acid peptides is fully washed, detached from the resin, and post-treated to yield a crude product. The crude product is dissolved, purified by preparative high-performance liquid chromatography twice, concentrated and lyophilized to obtain a pure product, and finally purified by a third HPLC purification to obtain a refined polypeptide product. This method not only ensures the efficiency of the synthesis, but also improves the purity of the product.

The steps for peptide synthesis (including elongating peptides from dipeptides to 29- or 17- or 13- amino acid peptides) are as follows:
1. Weigh 1.5 g of solid phase carrier Fmoc-wang-resin, pour into the glass sand core reaction column, add 5 mL of anhydrous DMF (dimethylformamide) to fully expand the resin for 2 hours, and remove the solvent DMF under reduced pressure.
a. De-capping: add 15 mL of de-capping solution (containing 20% pyridine, 80% DMF, in volume fraction). After a period of reaction, the de-capping solution is drained, washed once with 15 mL of de-capping solution, and the Fmoc protecting group is removed.
b. Washing: the de-capping solution was drained, and the resin was washed three times with 15 mL of DMF to thoroughly wash away the by-products.
c. Condensation: according to the pre-designated amino acid sequence of the polypeptide, the Fmoc-modified monomeric amino acid used for the peptide synthesis is dissolved in 5 mL of DMF, 0.5 mL DIEA (N,N-diisopropylethylamine) is added, and the reaction mix is added into a reaction vessel, let react for 2 hours under N2blow,removethe reaction solution by filtering, add 5 mL of methanol and perform blocking reaction for 1 hour,wash the resin three times with 15 mL of DCM (dichloromethane).
d. Washing:drain the reaction solution, wash the resin thoroughly with 15 mL of DMF to wash off by-products.
e. Detaching: the dried resin is placed in a round bottom flask, cleavage solutionis added to fully cleave the synthesized 29-peptide intermediate, and the resin is separated from the polypeptide by a sand core funnel, wherein the cleavage solution is composed of trifluoroacetic acid: phenol: water: thioanisole: EDT=90:3:3:2:2 by volume.

2. The post-treatment steps are as follows: first add anhydrous ether to the cleavage solutionto precipitate the polypeptide, then centrifuge, pour off the supernatant, then wash the polypeptide with anhydrous ether, and drain to obtain the crude peptide.
3. The steps for purification are as follows:
a. Dissolve: weigh the crude product into a 5-20 g/L solution and filter it with a 0.45 µm mixed filter.
Preparation: perform first purification, second purification and third purification through semi-preparative high-performance liquid chromatography to yield the refined polypeptides.The mobile phase is: phase A acetonitrile, phase B 0.1% TFA aqueous solution.
1)First purification: equilibrate the column with 30%-40% acetonitrile in water at a flow rate of 50 mL/min and rinse for 10 min. The crude products after filtering is loaded with an infusion pump at 1 mL/min.

**Table I. First Purification Elution Gradient**

| Time (min) | Flow rate (mL/min) | A% | B% | Wavelength nm |
|---|---|---|---|---|
| 0 | 60 | 10 | 90 | 220 |
| 30 | 60 | 30 | 70 | 220 |

Collect solutions with an absorption value greater than 200 mv at the wavelength of 220 nm, pool collected solution with a purity greater than 95% as a peak top, which is to be subjected to second purification.
2) Secondary purification: equilibrate a column with 30%-40% acetonitrile in water at a flow rate of 50 mL/min and rinse for 10 min.Thepeak top from the first purification was rotary evaporated to remove the organic solvent, and subsequently loaded with an infusion pump at 1 mL/min.

**Table 2. Second Purification Elution Gradient**

| Time (min) | Flow rate (mL/min) | A% | B% | Wave length nm |
|---|---|---|---|---|
| 0 | 60 | 5 | 95 | 220 |
| 30 | 60 | 20 | 80 | 220 |

Collect solutions with an absorption value greater than 200 mv at the wavelength of 220 nm, collect solution with a purity greater than 98% as the qualified solution.
b. Concentration, filtration, lyophilization: The qualified solution is concentrated under reduced pressure at 37°C using a rotary evaporator to remove residual solvent and injection water. Finally, filter through a 0.22 µm filter, and the filtrate was placed in a lyophilizing tray, and freeze-dried by a lyophilizer to obtain a pure product.
3)Third purification: equilibrate a column with 30%-40% acetonitrile in water at a flow rate of 50 mL/min and rinse for 10 min. Purify the samples obtained from the second purification with purity greater than 98% to obtain refined polypeptide.

**Table 3 Third Purification Elution Gradient**

| Time (min) | Flow rate (mL/min) | A% | B% | Wave length nm |
|---|---|---|---|---|
| 0 | 60 | 5 | 95 | 220 |
| 30 | 60 | 15 | 85 | 220 |

Collect solutions with an absorption value greater than 200 mv at the wavelength of 220 nm, pool the collected solution with a purity greater than 99.5% as the refined solution.
4. Purity testing. The purified product after lyophilization was collected, and the purity of the polypeptide was examined by analytical RP-HPLC. The analysis conditions were: mobile phase: CAN (+0.1% TFA), H2O (+0.1% TFA); acetonitrile (CAN) linear gradient: 15%-100%; flow rate: 1.5 mL/min; running time: 30 min; Sample size: 20 µL; detection wavelength: 220 nm.

In this experiment, the solid phase synthesis method was successfully used to synthesize the polypeptides. This method has high reproducibility, high operability, low pollution, and reversed-phase high-performance liquid chromatography for purification of peptides. Separation is enhanced using gradient elution compared to isocratic elution. The separation process has a reasonable retention time, high production efficiency, and high purity.

### Example 2

In the present embodiment, the polypeptides I-VI are synthesized by a liquid phase synthesis method, separated and purified by preparative HPLC, and the purity of the polypeptide is determined by an analytical HPLC. The following is the synthetic step of polypeptide I. The synthesis steps of polypeptide II-VI are the same as that of the polypeptide I.

The peptide I synthesis steps are as follows:
1. According to the sequence of polypeptide I, 1 mg of the first amino acid Pro and 1 mg of the second amino acid D-Pyr are linked by an amide bond in 10 mL of dichloromethane, and the inactive groups of the amino acid participating in the reaction is modified with Fmoc.
2. Add 10 mL of ammonia water to the above reaction system to remove the Fmoc group.
3. Repeat the reaction of the first step by adding the third amino acid D-Cys according to the sequence of the polypeptide I and link the third amino acid D-Cys with the dipeptide. The inactive group of the amino acid is modified with Fmoc.
4. Repeat steps 2 and 3 until the entire polypeptide sequence Pro-(D-Pyr)-(D-Cys)-Bip-Arg-Gly-Glu-Gly-Gly-Gly-Gly-Ile-Val-Arg-Arg-Ala-Asp-Arg-Ala-Ala-Val-Pro-Gly-Gly-Gly-Gly-Arg-Gly-Asp is synthesized.
5. Separate the polypeptide product by preparative HPLC. Mobile phase: linear gradient of acetonitrile: 15%-100%; flow rate: 1.5 mL/min; running time: 60 min; sample loading: 100 mL; detection wavelength: 220 nm.

### Example 3

### Establishment of an in vitro pulmonary fibrosis model

Human non-small cell lung cancer cells A549 were cultured in DMEM medium containing 10% (volume fraction) fetal bovine serum, cultured in a carbon dioxide incubator with a volume fraction of 5% at 37 °C, and changed once every other day, depending on cell density. A549 cells in logarithmic growth phase were digested with 0.25% trypsin to prepare a cell suspension, adjusted to a cell concentration of 1×109 cells/L, and seeded at a density of 4×105 cells/cm2 in a cell culture dish of 90 cm2. The cells were randomly divided into two groups: (1) control group: cultured in DMEM containing 10% (volume fraction) fetal bovine serum; (2) model group: containing 10% (volume fraction) fetal bovine serum, transforming growth factor (TGF-β1, final concentration 5 µg / L) was cultured in DMEM. The cells were cultured at 37 °C in a volumetric 5% carbon dioxide incubator, and changed every other day, depending on the cell density. Cell morphology was observed daily using an inverted microscope to determine when the model was established. When A549 cells are changed from the cobblestone shape of the original epithelial cells to the long spindle shape of the interstitial cells, the modeling is successfully established.

### Example 4

### Experimental procedure for treating pulmonary fibrosis with the fusion polypeptides

A549 cells not induced by TGF-β1 were used as the control group; the A549 cells induced by TGF-β1 and serving as the lung fibrosis model were further divided into multiple groups, one of which does not receive any drug and serves as a model group control, and the rest of the groups were administered with the polypeptides of different doses. After 48 hours of addition of the polypeptides, the cells of the control group, the model group, and the administration group were collected for subsequent measurement of molecular markers related to pulmonary fibrosis.

### Determination of hydroxyproline content in cells

Human specimen experiments show that a large amount of collagen accumulates in the late stage of pulmonary fibrosis, and the amount of collagen reflects the level of pulmonary fibrosis. Hydroxyproline accounts for 13.4% of collagen, so the content of hydroxyproline can reflect the accumulation of collagen during pulmonary fibrosis, which in turn reflects the degree of pulmonary fibrosis.

Prepare citric acid buffer: 120 g of sodium citrate, 12 mL of glacial acetic acid, 46 g of citric acid, 34 g of sodium hydroxide, dissolved in distilled water, adjust the pH to 6.0, and then dilute to 1000 mL.

Prepare 0.05 mol/L chloramine T solution: weigh 7.05 g of chloramine T, dissolve in 100 mL of distilled water, then add 150 mL of ethylene glycol, and add 250 mL of citric acid buffer to mix well.

Prepare p-dimethylaminobenzaldehyde: Reagent A: Take 20 mL of absolute ethanol, slowly add 2.74 mL of concentrated sulfuric acid; Reagent B: Take 12.0 g of p-dimethylaminobenzaldehyde, slowly add 40 mL of absolute ethanol. Heat in a water bath to completely dissolve and cool to room temperature, then slowly add the A solution to B and mix well.

Prepare a 3.5 mol/L perchloric acid solution: take 27 mL of perchloric acid and dilute to 100 mL with distilled water.

The specific steps are as follows: collect the cells in a 1 mL water solution and lyse the cells using a homogenizer; adjust the pH to neutral, dilute to 3 mL, add activated carbon to centrifuge; take 1 mL supernatant, add 1 mL solution 1 (1 mL citrate buffer and 1 mL 0.05mol/L chloramine T solution), stand for 10 minutes, add 1 mL of perchloric acid, stand for 5 minutes, add 1 mL of p-dimethylaminobenzaldehyde reagent, keep in 60 °C water bath for 15 minutes; centrifuge at 3500 rpm/minfor 10 minutes, take the supernatant to measure the absorbance value at a wavelength of 550 nm.

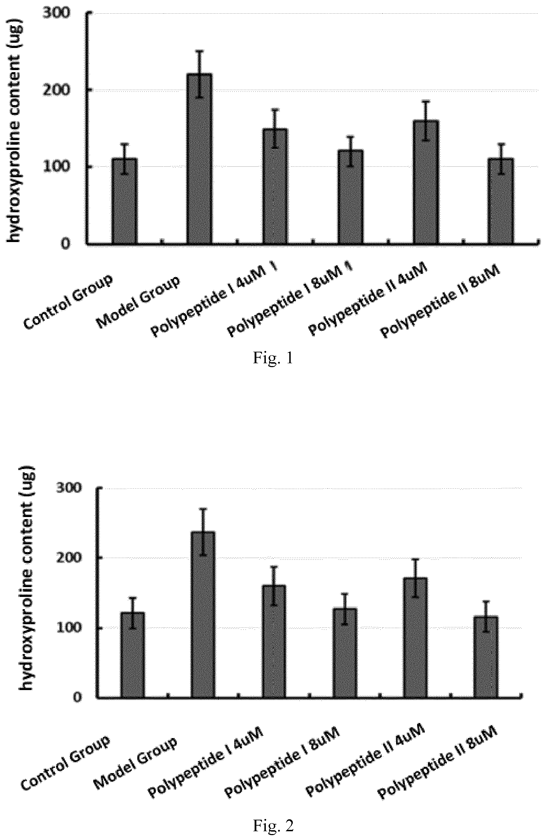

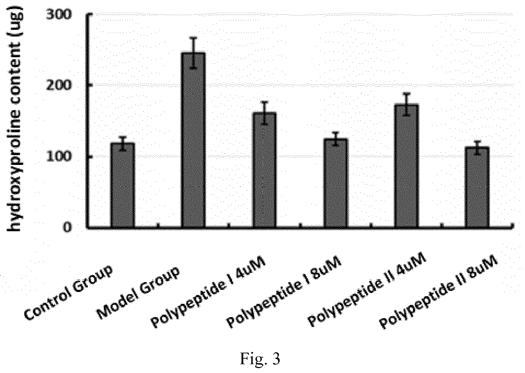

The test was repeated 3 times independently, and the inhibition rate was calculated as follows: inhibition rate (%) = (1 - experimental group absorbance / model group absorbance) × 100%. Table 4 shows the inhibition rate of the hydroxyproline content in the pulmonary fibrosis model of the polypeptides of the present invention. Figure 1 shows that polypeptides I and II of the present invention can inhibit the content of hydroxyproline in lung fibrosis model cells and inhibit the progression of pulmonary fibrosis; Figure 2 shows that polypeptide III and polypeptide IV of the present invention can inhibit the content of hydroxyproline in lung fibrosis model cells and inhibit the progression of pulmonary fibrosis; Figure 3 shows that the polypeptides V and VI of the present invention can inhibit the content of hydroxyproline in lung fibrosis model cells and inhibit the progression of pulmonary fibrosis.

**Table 4. Hydroxyproline Content Inhibition Rate in the Pulmonary Fibrosis Model by the Polypeptide of the Present Invention (%)**

| Concentration | Polypeptide I | Polypeptide II | PolypeptideI II | PolypeptideI V | Polypeptide V | Polypeptide VI |
|---|---|---|---|---|---|---|
| 4uM | 36.50 | 31.28 | 30.66 | 33.09 | 34.47 | 29.55 |
| 8uM | 52.14 | 57.35 | 56.21 | 47.28 | 49.25 | 54.17 |

### Example 5

### Inhibition of various lung infections by the polypeptide of the present invention

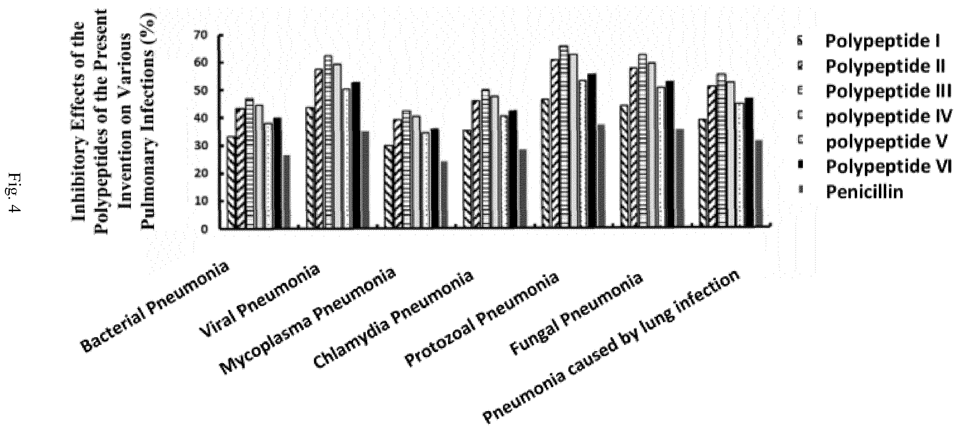

The mouse pneumonia model was successfully established by the nose-drop method. To build the mouse pneumonia model, BALB/C mice with a body mass of 16-22 g were selected and anesthetized pneumonia chain was prepared after anesthesia with ether on day 0, day 1, and day 2. Staphylococcal fluid, adenovirus concentrate, Mycoplasma pneumoniae, Chlamydia pneumoniae, protozoa, and pneumonia fungi were slowly dripped into the nasal cavity of the mice, so that they enter the tracheobronchial. During the operation process, care was exercised to prevent bacterial fluid from flowing into the esophagus, which leads to inactivation the bacterial fluid. After successful model establishment, the polypeptides of the present invention were administered, and the results were shown in Table 5. The polypeptide of the present invention displayed a more significant improvement on various lung infections than the penicillin-administered group (Figure. 4).The results were expressed in averagevalue ± standard deviation.

**Table 5. Inhibitory Effects of the Polypeptide of the Present Invention on Various Pulmonary Infections (%)**

| Type of Pneumonia | Polypeptide I | Polypeptide II | Polypeptide III | Polypeptide IV | Polypeptide V | Polypeptide VI | Penicillin |
|---|---|---|---|---|---|---|---|
| Bacterial | 33.15±10.06 | 43.43±12.38 | 47.07±13.26 | 44.75±13.08 | 38.12±11.17 | 39.78±9.86 | 26.52±11.07 |
| Viral | 43.78±14.17 | 57.35±16.34 | 62.17±17.51 | 59.10±18.42 | 50.35±15.73 | 52.54±13.88 | 35.02±15.58 |
| Mycoplasma | 29.89±12.97 | 39.16±11.16 | 42.44±11.96 | 40.35±16.87 | 34.37±14.40 | 35.87±12.71 | 23.91±14.27 |
| Chlamydia | 35.13±13.63 | 46.02±13.12 | 49.88±14.05 | 47.43±17.72 | 40.40±15.13 | 42.16±13.36 | 28.10±14.99 |
| Protozoal | 46.25±18.59 | 60.59±17.27 | 65.68±18.50 | 62.44±24.16 | 53.19±20.63 | 55.50±18.21 | 37.00±20.45 |
| Fungal | 43.91±15.11 | 57.52±16.39 | 62.35±17.56 | 59.28±19.64 | 50.50±16.77 | 52.69±14.80 | 35.13±16.62 |
| Pneumonia caused by lung infection | 38.79±16.78 | 50.81±14.48 | 55.08±15.52 | 52.37±21.81 | 44.61±18.63 | 46.55±16.44 | 31.03±18.46 |

### Example 6

### MTT assay for the inhibition of the growth of various lung cancer tumor cells by the polypeptide of the present invention

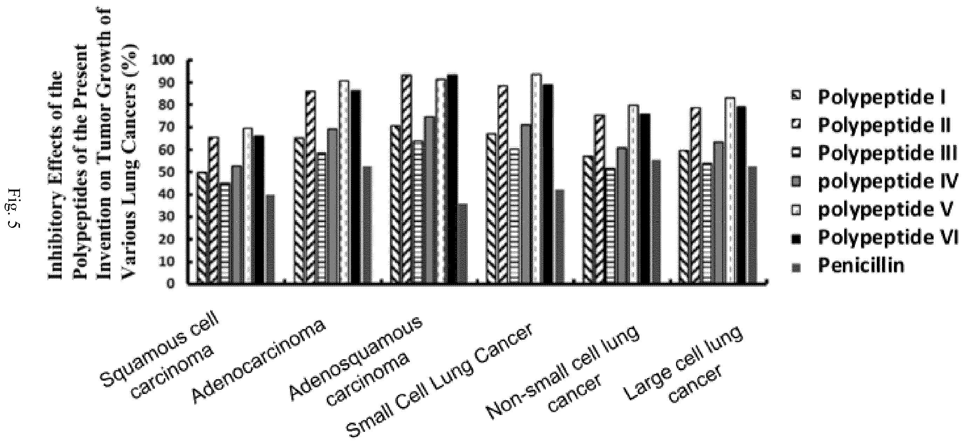

The inhibition of the growth of human lung cancer tumor cells by the polypeptide of the present invention was examined by MTT assay. The lung cancer tumor cells were cultured in a 37°C, 5% (volume fraction) CO2 incubator to a density of 90% or more, and collected by trypsinization. The cells were resuspended in the culture medium and counted under a microscope to adjust the cell concentration to 2.0. ×104 cells/mL, the cell suspension was inoculated into a 96-well plate at 100 µL per well, and cultured overnight at 37°C in a 5% (volume fraction) CO2 incubator. After the cells were completely adhered, the polypeptide of the present invention was added as a drug-administered group, and a culture solution containing no drug was used as a blank control group, and the culture solution was diluted to each predetermined concentration. Each dilution was separately added to a 96-well plate at 100 µL per well and incubated for 48 h at 37°C in a 5% (volume fraction) CO2 incubator. 20 µL of 5 mg/mL MTT was added to each well of a 96-well plate, and incubation was continued for 4 hours. The medium was aspirated and the cells were dissolved in 100 µL of DMSO per well. The absorbance was measured by a microplate reader at a detection wavelength of 570 nm and a reference wavelength of 630 nm, and the growth inhibition rate was calculated as follows: tumor growth inhibition rate (%) = (1 - administered group absorbance value / unadministered group absorbance value) x 100%, and the experiment was repeated 3 times independently. The results obtained by the test were expressed as mean ± standard deviation. The results in Table 6 indicate that the polypeptides of the present invention have a good inhibitory effect on proliferation of a variety of human lung cancer tumors (Figure 5).

**Table 6. Inhibitory Effects of the Polypeptidesof the Present Inventionon Tumor Growth of Various Lung CancersMeasured by MTTAssay (%)**

| Type of lung cancer | Polypeptide I | Polypeptide II | Polypeptide III | Polypeptide IV | Polypeptide V | Polypeptide VI | Docetaxel |
|---|---|---|---|---|---|---|---|
| Squamous cell carcinoma | 49.73±9.76 | 65.14±12.00 | 70.61±12.86 | 67.13±12.69 | 57.18±10.83 | 59.67±9.56 | 39.78±10.73 |
| Adenocarcinoma | 65.67±13.74 | 86.03±15.85 | 93.25±16.99 | 88.65±17.86 | 75.52±15.25 | 78.80±13.47 | 52.54±15.12 |
| Adenosquamous carcinoma | 44.84±12.58 | 58.73±10.82 | 63.67±11.60 | 60.53±16.36 | 51.56±13.97 | 53.80±12.33 | 35.87±13.84 |
| Small Cell Lung Cancer | 52.70±13.22 | 69.03±12.72 | 74.83±13.63 | 71.14±17.19 | 60.60±14.68 | 63.23±12.96 | 42.16±14.54 |
| Non-small cell lung cancer | 69.38±18.03 | 90.88±16.75 | 91.58±17.95 | 93.66±23.44 | 79.78±20.01 | 83.25±17.67 | 55.50±19.83 |
| Large cell lung cancer | 65.87±14.65 | 86.28±15.90 | 93.53±17.04 | 88.92±19.05 | 75.74±16.27 | 79.04±14.36 | 52.69±16.12 |

### Example 7

### MTT assay for the inhibition of the growth of tumor cells from a variety of different sources by the polypeptides of the invention

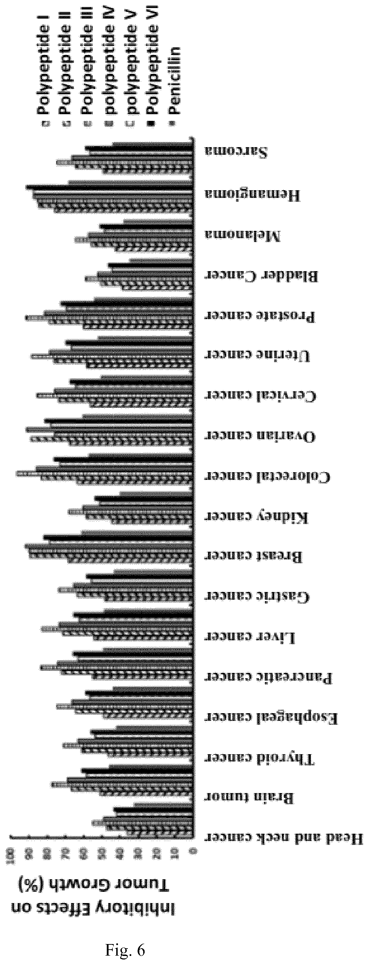

A variety of human tumor cells were cultured in a 37°C, 5% (volume fraction) CO2 incubator to a density of 90% or more, collected by trypsinization, resuspended in a culture medium and counted under a microscope, and the cells were harvested. The concentration was adjusted to 2.0 × 104 cells/mL, the cell suspension was inoculated into a 96-well plate at 100 µL per well and cultured overnight at 37°C in a 5% (volume fraction) CO2 incubator. After the cells were completely adhered, the polypeptides of the present invention were added as drug-administered groups, and a culture solution containing no drug was used as a blank control group, and the culture solution was diluted to each predetermined concentration. Each dilution was separately added to a 96-well plate at 100 µL per well and incubated for 48 hours at 37°C in a 5% (volume fraction) CO2 incubator. 20 µL of 5 mg/mL MTT was added to each well of a 96-well plate and incubation was continued for 4 h. The medium was aspirated and dissolved in 100 µL of DMSO per well. The absorbance was measured with a microplate reader at a detection wavelength of 570 nm and a reference wavelength of 630 nm, and the growth inhibition rate was calculated as follows: tumor growth inhibition rate (%) = (1 - absorbance value of the administered group / unadministered group absorbance value) x 100%, and the experiment was repeated 3 times independently. The results obtained from the test were expressed as mean ± standard deviation, and the tumor growth inhibition rate of the unadministered group was 0. The results in Table 7 indicate that the polypeptides of the present invention have a significant inhibitory effect on the growth of various tumors (Fig. 6).

**Table 7.Inhibitory Effects of the Polypeptides of the Present Invention on Various Tumor Growth Measured by MTTAssay (%)**

| Type of cancer | Polypeptide I | Polypeptide II | Polypeptide III | Polypeptide IV | Polypeptide V | Polypeptide VI | Docetaxel |
|---|---|---|---|---|---|---|---|
| Head and neck cancer | 36.22±10.06 | 47.44±11.27 | 55.05±12.07 | 48.89±13.08 | 41.65±11.17 | 43.46±9.86 | 32.59±9.86 |
| Brain tumor | 51.00±14.17 | 66.81±15.87 | 77.52±17.00 | 68.85±18.42 | 58.65±15.73 | 61.20±13.88 | 45.90±13.88 |
| Thyroid cancer | 46.70±12.97 | 61.18±14.53 | 70.99±15.57 | 63.05±16.87 | 53.71±14.40 | 56.04±12.71 | 42.03±12.71 |
| Esophageal cancer | 49.07±13.63 | 64.28±15.27 | 74.58±16.36 | 66.24±17.72 | 56.43±15.13 | 58.88±13.36 | 44.16±13.36 |
| Pancreatic cancer | 54.91±18.59 | 71.93±20.82 | 66.99±22.30 | 74.13±24.16 | 63.15±20.63 | 65.89±18.21 | 49.42±18.21 |
| Liver cancer | 54.38±15.11 | 71.24±16.92 | 82.66±18.13 | 73.42±19.64 | 62.54±16.77 | 65.26±14.80 | 48.95±14.80 |
| Gastric cancer | 48.41±16.78 | 63.41±18.79 | 73.58±20.14 | 65.35±21.81 | 55.67±18.63 | 58.09±16.44 | 43.57±16.44 |
| Breast cancer | 68.27±18.96 | 89.43±21.24 | 90.11±22.76 | 92.16±24.65 | 78.51±21.05 | 81.92±18.58 | 61.44±18.58 |
| Kidney cancer | 44.70±15.75 | 58.56±17.64 | 67.94±18.90 | 60.35±20.48 | 51.41±17.48 | 53.64±15.44 | 40.23±15.44 |
| Colorectal cancer | 63.55±17.65 | 83.25±19.77 | 96.60±21.18 | 85.80±22.95 | 73.08±19.60 | 76.26±17.30 | 57.20±17.30 |
| Ovarian cancer | 67.74±18.82 | 88.74±21.07 | 75.87±22.58 | 91.45±24.46 | 77.90±20.89 | 81.29±18.44 | 60.97±18.44 |
| Cervical cancer | 56.16±22.27 | 62.34±24.94 | 68.52±26.72 | 64.58±28.95 | 64.58±24.72 | 67.39±21.82 | 50.54±21.82 |
| Uterine cancer | 58.14±19.48 | 64.54±21.82 | 70.93±23.38 | 78.49±25.33 | 66.86±21.63 | 69.77±19.09 | 52.33±19.09 |
| Prostate cancer | 60.28±20.08 | 78.96±22.49 | 73.54±24.09 | 81.37±26.10 | 69.32±22.29 | 72.33±19.68 | 54.25±19.68 |
| Bladder Cancer | 38.69±10.75 | 50.68±12.04 | 58.81±12.90 | 52.23±13.97 | 44.49±11.93 | 46.43±10.53 | 34.82±10.53 |
| Melanoma | 42.41±11.78 | 55.55±13.19 | 64.46±14.14 | 57.25±15.31 | 48.77±13.08 | 50.89±11.54 | 38.17±11.54 |
| Hemangioma | 76.08±21.13 | 84.45±23.67 | 85.21±25.36 | 87.49±27.47 | 87.49±23.46 | 91.30±20.71 | 68.47±20.71 |
| sarcoma | 49.18±13.66 | 64.42±15.30 | 74.75±16.39 | 66.39±17.76 | 56.55±15.16 | 59.01±13.39 | 44.26±13.39 |

### Sequence Listing

<110>NANJING ANJI BIOLOGICAL TECHNOLOGY CO.,LTD
<120>MULTI-FUNCTIONAL FUSION POLYPEPTIDE, PREPARATION METHOD THEREOF, AND APPLICATION OF SAME
<160> 6
<170> Patent In version 3.3
<210> 1
   <211> 29
   <212> PRT
   <213>Artificial Sequences
<400> 1 wherein Pyr is 3-(3-pyridyl)-L-alanine and Bip is L-4, 4'-biphenylalanine;
<210> 2
   <211> 29
   <212> PRT
   <213>Artificial Sequences
<400> 2 wherein Pyr is 3-(3-pyridyl)-L-alanine and Bip is L-4, 4'-biphenylalanine;
<210> 3
   <211> 29
   <212> PRT
   <213>Artificial sequences
<400> 3 wherein Pyr is 3-(3-pyridyl)-L-alanine and Bip is L-4, 4'-biphenylalanine;
<210> 4
   <211> 29
   <212> PRT
   <213>Artificial sequences
<400> 4 wherein Pyr is 3-(3-pyridyl)-L-alanine and Bip is L-4, 4'-biphenylalanine;
<210> 5
   <211> 29
   <212> PRT
   <213>Artificial sequences
<400> 5 wherein Pyr is 3-(3-pyridyl)-L-alanine and Bip is L-4, 4'-biphenylalanine;
<210> 6
   <211> 29
   <212> PRT
   <213>Artificial sequences
<400> 6 wherein Pyr is 3-(3-pyridyl)-L-alanine and Bip is L-4, 4'-biphenylalanine.

### SEQUENCE LISTING

<110> NANJING ANJI BIOLOGICAL TECHNOLOGY CO.,LTD
<120> MULTI-FUNCTIONAL FUSION POLYPEPTIDE, PREPARATION METHOD THEREOF, AND APPLICATION OF SAME
<140> CNPCT/CN2017/075861
   <141> 2017-03-07
<150> CN2016101442130
   <151> 2016-03-14
<160> 6
<170> BiSSAP 1.3.6
<210> 1
   <211> 29
   <212> PRT
   <213> Artificial sequence
<220>
   <223> artificial sequence of MULTI-FUNCTIONAL FUSION POLYPEPTIDE
<220>
   <221> SITE
   <222> 2
   <223> Xaa is D-Pyr which is 3-(3-pyridyl)-L-alanine
<220>
   <221> SITE
   <222> 3
   <223> Xaa is D-Cys
<220>
   <221> SITE
   <222> 4
<223> Xaa is Bip which is L-4, 4'-biphenylalanine
<400> 1
   Pro Xaa Xaa Xaa Arg Gly Glu Gly Gly Gly Gly Ile Val Arg Arg Ala
   1               5                   10                  15
   Asp Arg Ala Ala Val Pro Gly Gly Gly Gly Arg Gly Asp
               20                  25
<210> 2
   <211> 29
   <212> PRT
   <213> Artificial sequence
<220>
   <223> artificial sequence of MULTI-FUNCTIONAL FUSION POLYPEPTIDE
<220>
   <221> SITE
   <222> 17
   <223> Xaa is D-Pyr which is 3-(3-pyridyl)-L-alanine
<220>
   <221> SITE
   <222> 18
   <223> Xaa is D-Cys
<220>
   <221> SITE
   <222> 19
   <223> Xaa is Bip which is L-4, 4'-biphenylalanine
<400> 2
   Ile Val Arg Arg Ala Asp Arg Ala Ala Val Pro Gly Gly Gly Gly Pro
   1               5                   10                  15
   Xaa Xaa Xaa Arg Gly Glu Gly Gly Gly Gly Arg Gly Asp
               20                  25
<210> 3
   <211> 29
   <212> PRT
   <213> Artificial sequence
<220>
   <223> artificial sequence of MULTI-FUNCTIONAL FUSION POLYPEPTIDE
<220>
   <221> SITE
   <222> 9
   <223> Xaa is D-Pyr which is 3-(3-pyridyl)-L-alanine
<220>
   <221> SITE
   <222> 10
   <223> Xaa is D-Cys
<220>
   <221> SITE
   <222> 11
   <223> Xaa is Bip which is L-4, 4'-biphenylalanine
<400> 3
   Arg Gly Asp Gly Gly Gly Gly Pro Xaa Xaa Xaa Arg Gly Glu Gly Gly
   1               5                   10                  15
   Gly Gly Ile Val Arg Arg Ala Asp Arg Ala Ala Val Pro
               20                  25
<210> 4
   <211> 29
   <212> PRT
   <213> Artificial sequence
<220>
   <223> artificial sequence of MULTI-FUNCTIONAL FUSION POLYPEPTIDE
<220>
   <221> SITE
   <222> 24
   <223> Xaa is D-Pyr which is 3-(3-pyridyl)-L-alanine
<220>
   <221> SITE
   <222> 25
   <223> Xaa is D-Cys
<220>
   <221> SITE
   <222> 26
   <223> Xaa is Bip which is L-4, 4'-biphenylalanine
<400> 4
   Arg Gly Asp Gly Gly Gly Gly Ile Val Arg Arg Ala Asp Arg Ala Ala
   1               5                   10                  15
   Val Pro Gly Gly Gly Gly Pro Xaa Xaa Xaa Arg Gly Glu
               20                  25
<210> 5
   <211> 29
   <212> PRT
   <213> Artificial sequence
<220>
   <223> artificial sequence of MULTI-FUNCTIONAL FUSION POLYPEPTIDE
<220>
   <221> SITE
   <222> 24
   <223> Xaa is D-Pyr which is 3-(3-pyridyl)-L-alanine
<220>
   <221> SITE
   <222> 25
   <223> Xaa is D-Cys
<220>
   <221> SITE
   <222> 26
   <223> Xaa is Bip which is L-4, 4'-biphenylalanine
<400> 5
   Ile Val Arg Arg Ala Asp Arg Ala Ala Val Pro Gly Gly Gly Gly Arg
   1               5                   10                  15
   Gly Asp Gly Gly Gly Gly Pro Xaa Xaa Xaa Arg Gly Glu
               20                  25
<210> 6
   <211> 29
   <212> PRT
   <213> Artificial sequence
<220>
   <223> artificial sequence of MULTI-FUNCTIONAL FUSION POLYPEPTIDE
<220>
   <221> SITE
   <222> 2
   <223> Xaa is D-Pyr which is 3-(3-pyridyl)-L-alanine
<220>
   <221> SITE
   <222> 3
   <223> Xaa is D-Cys
<220>
   <221> SITE
   <222> 4
   <223> Xaa is Bip which is L-4, 4'-biphenylalanine
<400> 6
   Pro Xaa Xaa Xaa Arg Gly Glu Gly Gly Gly Gly Arg Gly Asp Gly Gly
   1               5                   10                  15
   Gly Gly Ile Val Arg Arg Ala Asp Arg Ala Ala Val Pro
               20                  25

## Claims

1. A multifunctional fusion polypeptide, comprising domains of Pro-(D-Pyr)-(D-Cys)-Bip-Arg-Gly-Glu, Ile-Val-Arg-Arg-Ala-Asp-Arg-Ala-Ala-Val-Pro, Arg-Gly-Asp, and Gly-Gly-Gly-Gly.

2. A multifunctional fusion polypeptide according to claim 1, comprising at least one of the following sequences:
Peptide I: Pro-(D-Pyr)-(D-Cys)-Bip-Arg-Gly-Glu-Gly-Gly-Gly-Gly-Ile-Val-Arg-Arg-Ala-Asp-Arg-Ala-Ala -Val-Pro-Gly-Gly-Gly-Gly-Arg-Gly-Asp;
Peptide II: Ile-Val-Arg-Arg-Ala-Asp-Arg-Ala-Ala-Val-Pro-Gly-Gly-Gly-Gly-Pro-(D-Pyr)-(D-Cys)-Bip-Arg -Gly-Glu-Gly-Gly-Gly-Gly-Arg-Gly-Asp;
Peptide III: Arg-Gly-Asp-Gly-Gly-Gly-Gly-Pro-(D-Pyr)-(D-Cys)-Bip-Arg-Gly-Glu-Gly-Gly-Gly-Gly-Ile-Val -Arg-Arg-Ala-Asp-Arg-Ala-Ala-Val-Pro;
Peptide IV: Arg-Gly-Asp-Gly-Gly-Gly-Gly-Ile-Val-Arg-Arg-Ala-Asp-Arg-Ala-Ala-Val-Pro-Gly-Gly-Gly-Gly-Pro-(D-Pyr)-(D-Cys)-Bip-Arg-Gly-Glu;
Peptide V: Ile-Val-Arg-Arg-Ala-Asp-Arg-Ala-Ala-Val-Pro-Gly-Gly-Gly-Gly-Arg-Gly-Asp-Gly-Gly-Gly-Gly-Pro-(D-Pyr)-(D-Cys)-Bip-Arg-Gly-Glu;and
Peptide VI: Pro-(D-Pyr)-(D-Cys)-Bip-Arg-Gly-Glu-Gly-Gly-Gly-Gly-Arg-Gly-Asp-Gly-Gly-Gly-Gly-Ile-Val -Arg-Arg-Ala-Asp-Arg-Ala-Ala-Val-Pro;
wherein Pyr is 3-(3-pyridyl)-L-alanine and Bip is L-4, 4'-biphenylalanine.

3. A method for using the multifunctional fusion polypeptide of claim 1 or 2, comprising, using the multifunctional fusion polypeptide of claim 1 or 2 to generate medicines efficacious in treating human pulmonary fibrosis, pulmonary tissue lesions, lung cancer, and tumor.

4. A method according to claim 3, wherein the pulmonary tissue lesions include bacterial pneumonia, viral pneumonia, mycoplasmal pneumonia, chlamydia pneumonia, protozoal pneumonia and fungal pneumonia.

5. A method according to claim 3, wherein the lung cancer comprises squamous cell carcinoma, adenocarcinoma, glandular scale cancer, small cell lung cancer, non-small cell lung cancer, and large cell carcinoma.

6. A method according to claim 3, wherein the tumor comprises primary or secondary tumor, melanoma, hemangioma, and sarcoma originated from head, neck, brain, thyroid, esophagus, pancreas, liver, stomach, breast, kidney, gallbladder, colon or rectum, ovary, cervix, uterus, prostate, bladder or testis.

7. A method for preparing the multifunctional fusion polypeptide as disclosed in claim 1 or 2, wherein the multifunctional fusion polypeptide is synthesized via a solid phase method or a liquid phase method.

8. A method for preparing the multifunctional fusion polypeptide according to claim 7, wherein the solid phase method comprises the steps of:
selecting a Fmoc-wang-resin or Fmoc-CTC-resin as a starting material;
FMOC-protected amino acids are added one-at-a-time, according to the sequence of the fusion polypeptides, to yield a 29-amino acid peptide;
the 29-amino acid peptide is washed and detached from resin to yield a crude fusion polypeptide according to claim 1 or 2; and
the crude fusion polypeptide is dissolved, purified through a preparative high-performance liquid chromatography, and lyophilized to yield the fusion polypeptide according to claim 1 or 2.

9. A method for preparing the multifunctional fusion polypeptide according to claim 7, wherein the liquid phase method comprises the steps of:
sequentially connecting amino acids through amide bonds according to the fusion peptide sequence, wherein the inactive groups of the amino acids are protected by FMOC modification.
